# EUROPEAN PATENT SPECIFICATION

(11) **EP 3 241 521 B1**
(45) Date of publication and mention of the grant of the patent: **10.04.2019**
(21) Application number: 15831143.1
(22) Date of filing: 23.12.2015
(51) Int. Cl.: A61C 1/08, A61B 17/17

(54) **GUIDE FOR PLANNING AND DRILLING FOR THE SUBSEQUENT PLACEMENT OF DENTAL IMPLANTS**
FÜHRUNG ZUR PLANUNG UND BOHRUNG ZUR NACHTRÄGLICHEN POSITIONIERUNG VON ZAHNIMPLANTATEN
GUIDE DE PLANIFICATION ET DE PERCAGE POUR LE PLACEMENT ULTÉRIEUR D'IMPLANTS DENTAIRES

(30) Priority: 29.12.2014 ES 201431942
(43) Date of publication of application: 08.11.2017
(73) Proprietor: Phibo Dental Solutions, S.L., 08181 Sentmenat (Barcelona) (ES)
(72) Inventor: ALSINA FONT, Francesc, 08181 Sentmenat (Barcelona) (ES); LÓPEZ PÉREZ, Antonio, 08181 Sentmenat (Barcelona) (ES)
(74) Representative: Herrero & Asociados, S.L.
(86) International application number: PCT/ES2015/070943
(87) International publication number: WO 2016/107946

(56) References cited:
- WO-A1-94/00073
- WO-A2-98/43528
- JP-A- 2006 288 903
- US-A- 2 224 264
- US-A1- 2009 202 959
- US-A1- 2011 217 668

## Description

### Object of the invention

The object of the present invention is a guide for planning and drilling the subsequent placement of dental implants.
More specifically, the object of the drilling guide of the invention is to plan and guide the drilling of the bone tissue of a partially or completely edentulous patient in which a guide is needed for both depth and angle.

### Background of the invention

Implantology techniques currently enable dental roots to be substituted via implants, to which the corresponding prosthesis or artificial dental piece is in turn coupled.

Today the most widely used techniques are of special application when the patient only needs one dental piece in their denture to be substituted or replaced.

However, there are patients that have a completely toothless lower or upper jaw, or even both, either naturally or after the necessary extraction of all the dental pieces. In these cases, the substitution of each of these pieces by artificial implants becomes a complicated and arduous task that, as well as being very uncomfortable and taking a long time, which entails numerous and obvious discomfort for the patient, constitutes a high cost due to both the hours involved in surgery and the large amount of material used.

To this end, different techniques for rehabilitating edentulous patients appeared a few years ago, consisting of substituting all the pieces, generally 12 or 14, of the toothless jaw by only placing four implants in the front area of said jaw, where the density of the bone is greater and therefore the osseointegration has a greater chance of success.

Two of the four necessary implants are placed vertically and the other two in an inclined manner, specifically they are placed in the rear portion, i.e., at a certain angle with respect to the vertical implant. This is so because edentulous patients have usually suffered bone loss that makes it impossible to put the implants vertically along the sufficient length in order to obtain a minimum osseointegration that guarantees the load of the prosthesis and the angle is accurate in order to place a longer implant.

Two of the more well-known implants may be cited by way of example, which are known commercially as All-on-4® or Protocol™.

The advantages of this treatment with respect to using an implant for each one of the dental pieces to be substituted are clear, since the need for fewer implants means that discomfort is reduced for the patient, the time and stress of the surgery, the total time for treatment, as well as the cost, is reduced, to which the psychological benefits that enjoying a new denture entails must be added.

This inclination when drilling the bone tissue means that the surgeon must have some kind of guide element that enables him to orient the drilling in the most accurate way possible.

For this reason, for example in the All-on-4® technique, a guide is used consisting of a thin metal plate of considerable length and small in height to which a shaft or rod with a reduced diameter is joined at the centre point thereof. This rod is introduced in a small hole performed by the surgeon in the central point of the front portion of the jaw of the patient such that, once inserted, the metal plate may be bent or moulded in order to adapt to the contour of the bone ridge of the patient.

Said plate also has a series of vertical marks uniformly distributed along the entire length thereof that enable, when performing the angled drilling, the surgeon to have a reference for the drilling inclination using the diagonal between the marks as a reference.

This technique however, has notable drawbacks. Firstly, the lack of accuracy that is provided may be cited, since although the surgeon may guide the drilling using the vertical marks of the plate as a reference, said drilling still depends on the skill of said surgeon to first drill with the appropriate angle and then maintain the inclination during drilling. Furthermore, there is a high risk of involuntary movement in both the drill and the plate itself, which in the case of the latter tends to rotate about the vertical axis that constitutes the rod thereof, which causes the drilling to be performed with the incorrect inclination.

Secondly, there is the drawback of having to perform additional drilling, however small it may be, in the mouth of the patient, which constitutes added discomfort to the stress of the surgery.

Lastly, another drawback of this solution is that the depth of the drilling is not controlled, thus making it difficult to plan in advance on a model before the surgery.

In the state of the art there are examples of guides for drilling the bone tissue of a patient with the object of subsequently placing a dental implant, whether it is the first drilling, such as in US6062856, or if there is already a first hole and other holes are to be performed at a certain distance or specific position with respect to said first hole. Examples of this last case may be seen, for example, in US2224264, US5741133, US8821159 or WO200134055.

However, the guides shown in these documents do not have the possibility of guiding the drilling at an angle, thus meaning they are not useful for this purpose.

### Description of the invention

The guide for planning and drilling dental implants of the present invention resolves the aforementioned problems of the state of the art and constitutes a tool that is simple to use, versatile, adaptable and reusable, which guides the drilling of the bone tissue of the patient both in terms of angle and depth without the need to perform additional drilling that is not going to be used afterwards as a hole for the corresponding implant.

To this end, the planning and drilling guide of the invention is according to claim 1.

Thus, once a first perforation that does not need to be angled, i.e. a vertical hole in the bone tissue located in the front portion of the mouth of the patient, has been performed the surgeon then inserts the rod of the drilling guide of the invention into said vertical hole.

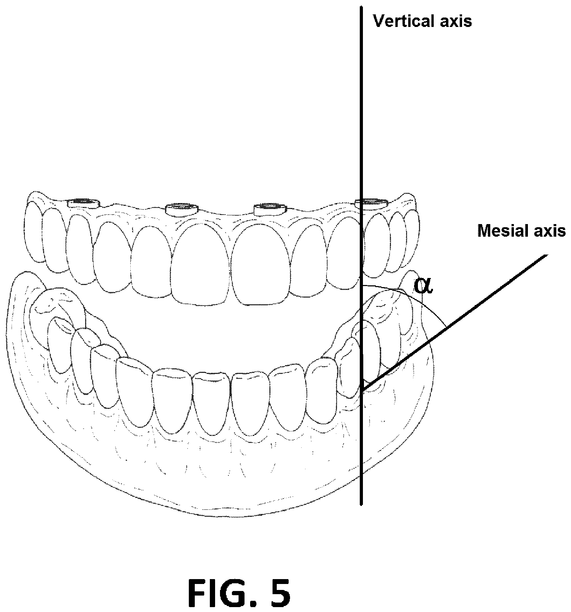

Clearly, said surgeon will have previously selected the appropriate plate, i.e., the one in which the plate has the appropriate angle at the end thereof, and that may be from 0° to 60°, the angle α being that which is comprised between the vertical plane of the bone tissue and the axis of the angle towards the mesial, as may be schematically seen in figure 5.

Once the rod has been inserted in said vertical hole and said hole is secured due to the retention means, the surgeon positions the plate in the appropriate position due to the rotation means such that the body of the guide is placed so that it correspond to the area of the bone tissue where the angled drilling is to be performed.

Specifically, the angled guiding of the drill is carried out due to the fact that the guide body is positioned on the angled end of the plate, meaning that the surgeon must simply pass the drill through said guide body in order for it to be correctly inclined. Furthermore, said guide body is such that it only enables the drill bit to enter, constituting a stop so that the drill cannot advance and the hole is performed at the correct depth. More specifically, the length of the drills and the depth of the hole to be achieved in the bone tissue being known, the guide body has a height such that, as mentioned above, it constitutes a stop in order to prevent the advance of the drill once the necessary depth has been drilled.

At the same time, as well as the rotation means that enable the relative movement between the plate and the rod, said plate further comprises horizontal movement means formed by an elongated opening extending between the free end where the guide body is located and the free end opposite to the guide body that enable the distance of the angled end of said plate to be varied with respect to said rod. That is to say, that through said horizontal movement means it is possible to regulate the distance from the rod, and therefore from the vertical hole of the bone tissue in which said rod is inserted, to the guide body through which the drill bit is inserted with the necessary angle and depth.

Therefore, once the guide of the invention is positioned in the vertical hole resulting from a first perforation of the bone tissue of the patient through the rod and this being fixed to said tissue due to the retention means, the surgeon may position the guide body on any point of the mouth of the patient within a circular surface with the centre in said vertical hole and with a variable radius that may be selected through the horizontal movement means of the plate.

The advantages of the drilling guide of the invention are easily discerned from this description, since it enables the spatial placement of the implants to be planned, a secure and easy guide for drilling, both in terms of angle and depth, which facilitates the task of the surgeon as well as minimising errors during the drilling operation. Another advantage is that one of the vertical drill holes for placing one of the implants is used as a positioner for the guide and does not need additional perforations in the mouth of the patient, which means it is a less traumatic treatment for the patient.

### Description of the drawings

As a complement to the description provided herein, and for the purpose of helping to make the characteristics of the invention more readily understandable, in accordance with a preferred practical embodiment thereof, said description is accompanied by a set of drawings constituting an integral part of the same, which by way of illustration and not limitation represent the following:
- Figure 1 shows a perspective view of the drilling guide of the present invention.
- Figure 2 shows an elevation view of the drilling guide of the present invention.
- Figure 3 shows an exploded perspective view of the elements that make up the drilling guide of the present invention.
- Figure 4 shows an exploded perspective view of some of the elements of figure 3 according to a possible embodiment of the invention.
- Lastly, figure 5 shows a schematic representation of the mouth of a patient in which the angle comprised between the vertical of the bone tissue plane and the axis angle towards the mesial is represented.

### Preferred embodiments of the invention

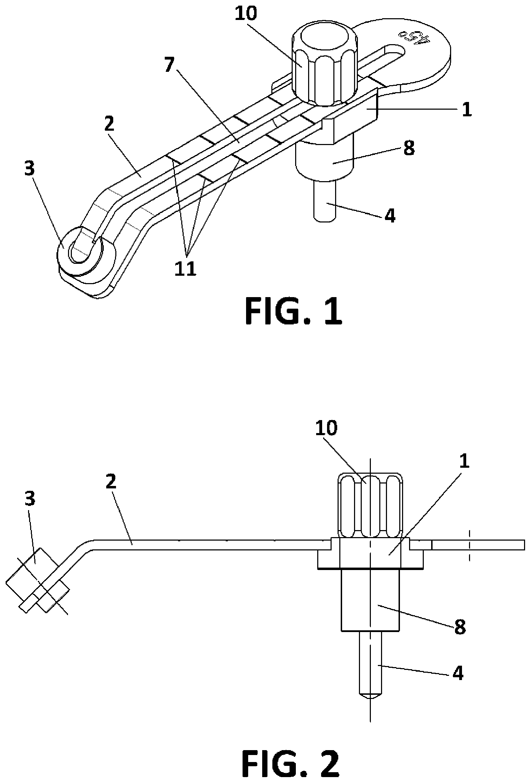

As may be seen in figures 1 to 5, in which a preferred embodiment of the invention is shown, the guide for planning and drilling dental implants is structured such that it comprises:
- A support (1);

A plate (2) in the form of a horizontal sheet that is fastened on the support (1) but may move and be detached with respect to it, and is therefore interchangeable and which comprises an angle at at least one of the ends thereof.

Furthermore, according to a possible embodiment of the invention, said plate (2) comprises, at at least one of the ends thereof, a means for regulating the angle (not shown) such that different angles for drilling the bone tissue may be selected using a single plate, thus removing the need to use different plates for each one of the angles. Said means for regulating the angle may be formed by, for example, a hinge or similar that, alongside the blocking means, enable the position of said end of the plate to be positioned and fixed at will or in predetermined positions and angles α, from 0° to 60°.
- A guide body (3) located at the angled end of the plate (2) for correctly guiding said drill bit or drill responsible for perforating the bone tissue with the appropriate angle and depth.

According to a possible practical embodiment shown in the figures, said guide body (3) is made up of a cylindrical body that has a wide radial slot to facilitate the insertion of the drill in the case of compromised openings.

Furthermore, according to another possible embodiment of the invention, said guide body (3) may be detached and is interchangeable, thus enabling it to be substituted for another depending on the diameter and length of the drill, equipping the guide of the invention with even greater versatility.
- A rod (4), preferably cylindrical, with one upper end and one lower end, wherein:
   ∘ The upper end is joined to the support (1) through rotation means (5) that enable the relative movement between both.

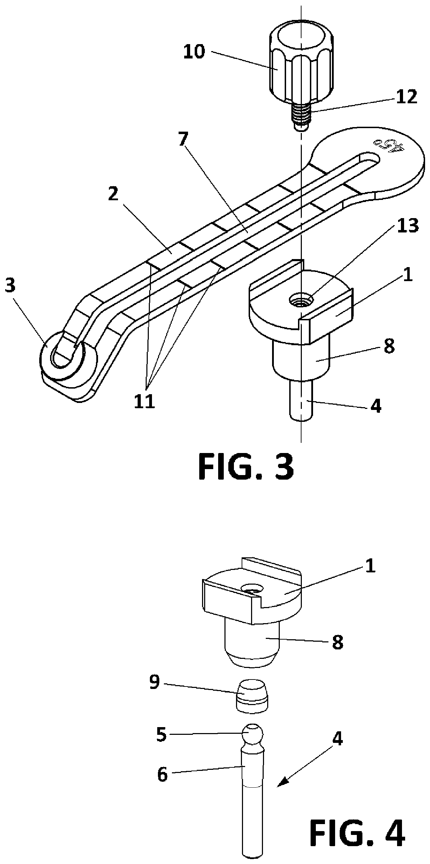

According to a possible practical embodiment, shown in figure 4, said rotation means (5) are formed by a ball-shaped extremity that cooperates with a spherical cavity in the support (1) such that said support (1) may rotate freely on the upper end of the rod (4), facilitating the placement and orientation thereof.
∘ The lower end is inserted in the bone tissue of the mouth of the patient, fastening thereto due to retention means (6).

According to a possible practical embodiment of the invention shown in figure 4, said retention means (6) are constituted by a conical section that increases in diameter as it moves away from the lower end, such that adjustment between the rod (4) and the bone tissue is carried out by friction.

According to another possible embodiment, said conical section may also comprise a certain surface roughness obtained by means of any method known with the aim of improving the fastening between the rod (4) and bone tissue.

Furthermore, and as may be seen in the figures, especially in numbers 1 and 3, the plate (2) comprises horizontal movement means (7) that enable the distance between the rod (4) and the guide body (3) located at the angled end of said plate (2) to be varied.

According to a possible embodiment of the invention shown in said figures, the horizontal movement means are formed by an elongated opening (7) extending between the free end where the guide body is located and the free end opposite to the guided body, such that the assembly formed by the support (1) and the rod (4) can move along the opening (7) in order to be able to vary the distance between the rod (4) and the guide body (3). Specifically, in said exemplary embodiment, the movement is achieved due to the effects of a tightening element (10) that fastens the support (1) to the plate (2) by means of a second rod (12) or similar that is inserted through said elongated opening (7) in a hole (13) in the upper portion of the support (1).

Furthermore, it is possible to facilitate the insertion of the drill, in the cases where the openings are compromised, due to said elongated opening (7), which runs to the guide body (3). In other words, the elongated opening (7) or groove therefore has a dual function, constituting of, on the one hand, horizontal movement means and, on the other hand, constituting a hole to facilitate the insertion of the drill when necessary thanks to the wide radial slot of the guided body (3) is facing the opening (7).

Furthermore, as may be seen in the figures, the support (1) has a lower body (8) that serves to house both the second rod (12) of the tightening element (10) and the upper end of the rod (4), thus meaning that in the case in which said upper end has a ball, it has a complementary spherical cavity.

Moreover, according to a possible practical embodiment shown in figures 1 and 3, the plate (2) has position markers (11) such that the surgeon may have a reference of the relative movement between the support (1) and said plate (2).

Nevertheless, according to a possible practical embodiment of the invention, shown in figure 4, said upper end of the rod (4) may be joined to the support (1) by means of an intermediate element (9) that is housed in the lower body (8) of said support (1).

Lastly, the guide of the invention is embodied with any material that fulfils the mechanical and biocompatibility requirements and which may be sterilised, such as metals like stainless steel or titanium, or plastics.

## Claims

1. A guide for planning and drilling the subsequent placement of dental implants, comprising:
- A support (1);
- A plate (2) fastened on the support (1) such that the plate (2) can be moved and detached with respect to the support (1);
- A rod (4) comprising an upper end that is rotatably joined to the support (1) that enable the relative movement between them, and a lower end that is insertable in the bone tissue of the mouth of the patient,
wherein the plate (2) comprises:
- a guide body (3) located at one of its free end; and
- horizontal movement means formed by an elongated opening (7) extending between the free end where the guide body is located and the free end opposite to the guided body , such that the assembly formed by the support (1) and the rod (4) can move along the opening (7) in order to be able to vary the distance between the rod (4) and the guide body (3),
wherein the guided body (3) is made up of a cylindrical body that has a wide radial slot facing the opening (7) such as to facilitate the insertion of the drill in the guided body (3); **characterized in that** the free end of the plate (2) where the guide body (3) is located forms an angle relative to the rest of the plate, and the guide body (3) also constitutes a drill stop, so that the drill can be inserted into the bone tissue with the appropriate angle and depth.

2. The guide for planning and drilling dental implants according to claim 1, **characterised in that** the plate (2) comprises a means for regulating the angle such that different angles for drilling the bone tissue can be selected using a single plate.

3. The guide for planning and drilling dental implants according to any of the preceding claims, **characterised in that** it comprises a tightening element (10) that fastens the support (1) to the plate (2) by means of a second rod (12) that is complementary with a hole (13) of the support (1).

4. The guide for planning and drilling dental implants according to any of the preceding claims, **characterised in that** the rotatably joint between the rod (4) and the support (1) is enabled by rotation means (5) formed by a ball-shaped extremity at the upper end of the rod (4) that cooperates with a spherical cavity of the support (1).

5. The guide for planning and drilling dental implants according to any of the preceding claims, **characterised in that** the lower end of the rod (4) comprises retention means (6) to fasten it to the bone tissue.

6. The guide for planning and drilling dental implants according to claim 5, **characterised in that** the retention means (6) comprise a conical section that increases in diameter as it moves away from the lower end.

7. The guide for planning and drilling dental implants according to claim 5 or 6, **characterised in that** the retention means (6) comprise a rough surface of the lower end of the rod (4).

8. The guide for planning and drilling dental implants according to any of the preceding claims, **characterised in that** the support (1) has a lower body (8) that serves as a housing for the upper end of the rod (4).

9. The guide for planning and drilling dental implants according to any of the preceding claims, **characterised in that** it comprises an intermediate element (9) through which the upper end of the rod (4) is joined to the support (1).

10. The guide for planning and drilling dental implants according to any of the preceding claims, **characterised in that** the plate (2) has position markers (11).

11. The guide for planning and drilling dental implants according to any of the preceding claims, **characterised in that** the guide body (3) can be removed or interchanged.

12. The guide for planning and drilling dental implants according to any of the preceding claims, **characterised in that** it is embodied with any material that fulfils the mechanical and biocompatibility requirements and which can be sterilised, such as metals like stainless steel or titanium, or plastics.

## Patentansprüche

1. Führung zum Planen und Bohren der nachträglichen Positionierung von Zahnimplantaten, mit:
- einer Halterung (1);
- einer Platte (2), die an der Halterung (1) derart befestigt ist, dass die Platte (2) bewegbar und von der Halterung (1) abnehmbar ist;
- einem Stab (4) mit einem oberen Ende, das drehbar mit der Halterung (1) verbunden ist, so dass die relative Bewegung zwischen ihnen möglich ist, und mit einem unteren Ende, das in das Knochengewebe des Mundes des Patienten einführbar ist,
wobei die Platte (2) aufweist:
- einen Führungskörper (3), der an einem ihrer freien Enden angeordnet ist; und
- eine Horizontalbewegungseinrichtung, die durch eine längliche Öffnung (7) gebildet ist, die zwischen dem freien Ende, an welchem der Führungskörper angeordnet ist, und dem freien Ende, das dem Führungskörper gegenüber liegt, verläuft derart, dass die durch die Haltung (1) und den Stab (4) gebildete Anordnung entlang der Öffnung (7) bewegbar ist, um in der Lage zu sein, den Abstand zwischen dem Stab (4) und dem Führungskörper (3) zu variieren,
wobei der Führungskörper (3) als ein Zylinderkörper aufgebaut ist, der einen weiten radialen Schlitz hat, der der Öffnung (7) zugewandt ist, derart, dass das Einführen des Bohrers in den Führungskörper (3) ermöglicht wird;
**dadurch gekennzeichnet, dass**
das freie Ende der Platte (2), an welchem der Führungskörper (3) angeordnet ist, einen Winkel in Bezug auf den Rest der Platte bildet, und der Führungskörper (3) auch einen Bohranschlag bildet, so dass der Bohrer in das Knochengewebe mit einem geeigneten Winkel und einer geeigneten Tiefe einführbar ist.

2. Führung zum Planen und Bohren von Zahnimplantaten nach Anspruch 1, **dadurch gekennzeichnet, dass** die Platte (2) eine Einrichtung zum Steuern des Winkels derart aufweist, dass unterschiedliche Winkel zum Bohren des Knochengewebes unter Anwendung einer einzigen Platte auswählbar sind.

3. Führung zum Planen und Bohren von Zahnimplantaten nach einem der vorhergehenden Ansprüche, **dadurch gekennzeichnet, dass** sie ein Befestigungselement (10) aufweist, das die Halterung (1) an der Platte (2) mittels eines zweiten Stabs (12) befestigt, der komplementär zu einer Bohrung (13) der Halterung (1) ist.

4. Führung zum Planen und Bohren von Zahnimplantaten nach einem der vorhergehenden Ansprüche, **dadurch gekennzeichnet, dass** die drehbare Verbindung zwischen dem Stab (4) und der Halterung (1) durch eine Dreheinrichtung (5) ermöglich wird, die durch eine kugelförmige Extremität an dem oberen Ende des Stabs (4) gebildet ist, die mit einem kugelförmigen Hohlraum der Halterung (1) zusammenwirkt.

5. Führung zum Planen und Bohren von Zahnimplantaten nach einem der vorhergehenden Ansprüche, **dadurch gekennzeichnet, dass** das untere Ende des Stabs (4) eine Rückhalteeinrichtung (6) aufweist, um ihn an dem Knochengewebe zu befestigen.

6. Führung zum Planen und Bohren von Zahnimplantaten nach Anspruch 5, **dadurch gekennzeichnet, dass** die Rückhalteeinrichtung (6) einen konischen Abschnitt aufweist, der mit zunehmender Entfernung von dem unteren Ende im Durchmesser zunimmt.

7. Führung zum Planen und Bohren von Zahnimplantaten nach Anspruch 5 oder 6, **dadurch gekennzeichnet, dass** die Rückhalteeinrichtung (6) eine raue Oberfläche des unteren Endes des Stabs (4) aufweist.

8. Führung zum Planen und Bohren von Zahnimplantaten nach einem der vorhergehenden Ansprüche, **dadurch gekennzeichnet, dass** die Halterung (1) einen unteren Körper (8) hat, der als Gehäuse für das obere Ende des Stabs (4) dient.

9. Führung zum Planen und Bohren von Zahnimplantaten nach einem der vorhergehenden Ansprüche, **dadurch gekennzeichnet, dass** sie ein Zwischenelement (9) aufweist, durch welches das obere Endes des Stabs (4) mit der Halterung (1) verbunden ist.

10. Führung zum Planen und Bohren von Zahnimplantaten nach einem der vorhergehenden Ansprüche, **dadurch gekennzeichnet, dass** die Platte (2) Positionsmarkierungen (11) aufweist.

11. Führung zum Planen und Bohren von Zahnimplantaten nach einem der vorhergehenden Ansprüche, **dadurch gekennzeichnet, dass** der Führungskörper (3) entnehmbar oder austauschbar ist.

12. Führung zum Planen und Bohren von Zahnimplantaten nach einem der vorhergehenden Ansprüche, **dadurch gekennzeichnet, dass** sie mit einem Material aufgebaut ist, das die Anforderungen hinsichtlich der Mechanik und Biokompatibilität erfüllt und das sterilisierbar ist, etwa Kunststoffe oder Metalle wie Edelstahl oder Titan.

## Revendications

1. Guide de planification et de perçage pour le placement ultérieur d'implants dentaires comprenant :
un support (1) ;
une plaque (2) fixée sur le support (1) de sorte que la plaque (2) peut être déplacée, et détachée par rapport au support (1) ;
une tige (4) ayant une extrémité supérieure qui est assemblée, de manière rotative, au support (1) qui permet le mouvement relatif entre eux, et une extrémité inférieure qui peut être insérée dans le tissu osseux de la bouche du patient,
dans lequel la plaque (2) comprend :
un corps de guidage (3) positionné au niveau de l'une de ses extrémités libres ; et
un moyen de déplacement horizontal formé par une ouverture allongée (7) s'étendant entre l'extrémité libre où le corps de guidage est positionné et l'extrémité libre opposée au corps de guidage, de sorte que l'ensemble formé par le support (1) et la tige (4) peut se déplacer le long de l'ouverture (7) afin de pouvoir modifier la distance entre la tige (4) et le corps de guidage (3),
dans lequel le corps guidé (3) et composé d'un corps cylindre qui a une large fente radiale faisant face à l'ouverture (7) afin de faciliter l'insertion du foret dans le corps guidé (3) ;
**caractérisé en ce que** l'extrémité libre de la plaque (2) où le corps de guidage (3) est positionné, forme un angle par rapport au reste de la plaque, et le corps de guidage (3) constitue également un arrêt de foret, de sorte que le foret peut être inséré dans le tissu osseux avec l'angle et la profondeur appropriés.

2. Guide de planification et de perçage d'implants dentaires selon la revendication 1, **caractérisé en ce que** la plaque (2) comprend un moyen pour réguler l'angle, de sorte que différents angles pour percer le tissu osseux peuvent être sélectionnés à l'aide d'une seule plaque.

3. Guide de planification et de perçage d'implants dentaires selon l'une quelconque des revendications précédentes, **caractérisé en ce qu'**il comprend un élément de serrage (10) qui fixe le support (1) sur la plaque (2) au moyen d'une seconde tige (12) qui est complémentaire avec un trou (13) du support (1).

4. Guide de planification et de perçage d'implants dentaires selon l'une quelconque des revendications précédentes, **caractérisé en ce que** le joint rotatif entre la tige (4) et le support (1) est autorisé par un moyen de rotation (5) formé par une extrémité en forme de rotule au niveau de l'extrémité supérieure de la tige (4) qui coopère avec une cavité sphérique du support (1).

5. Guide de planification et de perçage d'implants dentaires selon l'une quelconque des revendications précédentes, **caractérisé en ce que** l'extrémité inférieure de la tige (4) comprend un moyen de retenue (6) pour la fixer sur le tissu osseux.

6. Guide de planification et de perçage d'implants dentaires selon la revendication 5, **caractérisé en ce que** le moyen de retenue (6) comprend une section conique qui augmente du point de vue au diamètre au fur et à mesure qu'elle s'éloigne de l'extrémité inférieure.

7. Guide de planification et de perçage d'implants dentaires selon la revendication 5 ou 6, **caractérisé en ce que** le moyen de retenue (6) comprend une surface rugueuse de l'extrémité inférieure de la tige (4).

8. Guide de planification et de perçage d'implants dentaires selon l'une quelconque des revendications précédentes, **caractérisé en ce que** le support (1) a un corps inférieur (8) qui sert de logement pour l'extrémité supérieure de la tige (4).

9. Guide de planification et de perçage d'implants dentaires selon l'une quelconque des revendications précédentes, **caractérisé en ce qu'**il comprend un élément intermédiaire (9) par le biais duquel l'extrémité supérieure de la tige (4) est assemblée au support (1).

10. Guide de planification et de perçage d'implants dentaires selon l'une quelconque des revendications précédentes, **caractérisé en ce que** la plaque (2) a des marqueurs de position (11).

11. Guide de planification et de perçage d'implants dentaires selon l'une quelconque des revendications précédentes, **caractérisé en ce que** le corps de guidage (3) peut être retiré ou interchangé.

12. Guide de planification et de perçage d'implants dentaires selon l'une quelconque des revendications précédentes, **caractérisé en ce qu'**il est mis en oeuvre avec n'importe quel matériau qui satisfait les exigences mécaniques et de biocompatibilité et qui peut être stérilisé, tel que les métaux comme l'acier inoxydable ou le titane, ou bien le plastique.
